# EUROPEAN PATENT SPECIFICATION

(11) **EP 0 512 744 B1**
(45) Date of publication and mention of the grant of the patent: **26.06.1996**
(21) Application number: 92303870.7
(22) Date of filing: 29.04.1992
(51) Int. Cl.: A61K 7/06, A61K 7/08

(54) **Hair treatment composition containing a perfluoropolyether compound and a pearlescing agent**
Haarbehandlungsmittel mit Perfluoropolyethern und einem Perlglanzmittel
Composition pour le traitement des cheveux contenant un perfluoropolyether et un agent nacre

(30) Priority: 03.05.1991 GB 9109693
(43) Date of publication of application: 11.11.1992
(73) Proprietor: UNILEVER PLC, London EC4P 4BQ (GB); UNILEVER N.V., NL-3013 AL Rotterdam (NL)
(72) Inventor: Murray, Andrew Malcolm, Parkgate, South Wirral L64 6TL (GB)
(74) Representative: Mulder, Cornelis Willem Reinier, Dr.

(56) References cited:
- EP-A- 0 034 846
- EP-A- 0 360 292
- EP-A- 0 390 206
- EP-A- 0 486 135
- WO-A-88/06434
- PATENT ABSTRACTS OF JAPAN vol. 12, no. 349 (C-529)(3196) 20 September 1988

## Description

The present invention relates to hair-treatment compositions and more particularly to shampoos or conditioning compositions which comprise a perfluoropolyether material in a pearlescing system.

It is known that oils can provide conditioning benefits such as ease of combing of wet or dry hair, and tactile improvements such as softness and improved shine. Generally, however, these materials are only effective at high levels.

The use of perfluoropolyethers of the formula

F(C₃F₆O-)ₙ-C₂F₅

where n is from 4 to 500, in cosmetics is disclosed in JP 63107911 (Shiseido).

EP-A-360292 (Ausimont) relates to the use of 0.01 to 20% by weight of perfluoropolyethers having perfluoroalkyl end groups in foam baths, cleansing milks, bath oils and liquid soaps for the treatment of seborrhea.

EP-A-390206 relates to stable cosmetic and dermatological emulsions comprising perfluoropolyethers having perfluoroalkyl end groups. Only high levels of PFPE materials, i.e. in excess of 0.01% by weight, are disclosed.

EP-A-0486135 which is prior art according to Art. 54(3) EPC, relates to shampoos or conditioning compositions comprising very low levels of perfluoropolyethers. Pearlescing systems are not disclosed.

WO-A-8806434 relates to conditioning compositions comprising fluorosurfactants present at very low levels. There is no disclosure of a pearlescing system and the perfluoropolyethers.

What we have surprisingly found is that the shine and softness of hair, i.e. its condition, can be improved using a hair-treatment composition which contains low levels of perfluoropolyether materials in a pearlescing system. In particular it has been found that such conditioning benefits can be provided by employing a hair-treatment composition which comprises from about 0.00001 to about 0.01% by weight of a perfluoropolyether material in a pearlescing system.

Further advantages obtained by utilising perfluoropolyether in pearlescing systems are that such systems are thought to be more versatile in terms of surfactant selection relative to non-pearlescing systems which require a far more careful selection of surfactant with which to suspend perfluoropolyether materials. Also, it is considered that pearlescing systems help to further improve the tactile performance of hair care products containing perfluoropolyether materials even when the perfluoropolyether material is present at very low levels.

According to the present invention there is provided a hair treatment composition comprising: (i) from about 0.00001 to about 0.01% by weight of a perfluoropolyether material; and (ii) a suitable pearlescing agent, characterised by:
(a) having higher dissolution and melting points than normal hair care composition storage temperatures; and (b) forming thin platelet-type crystals in the hair treatment composition.

Preferably the hair treatment composition according to the invention is a shampoo or conditioning shampoo composition.

The invention will now be described in detail.

### Perfluoropolyether

The hair-treatment composition of the invention comprises a perfluoropolyether material. Suitable perfluoropolyethers and their method of preparation are described in GB 1,104,482, US 3,242,218, US 3,665,041, US 3,715,378, US 4,523,039, EP 148,482 and EP 191,490. Preferred perfluoropolyether materials are homo-or copolymers of perfluoropolyether materials of the following formula:

F-(C_{y}F_{2y}O)ₙ-C_{z}F_{(2z+1)}

wherein
z is an integer from 1 to 6, more preferably 1-3, most preferably 1 or 2;
for each monomer, Y is independently selected from the integer-range from 1 to 6, more preferably 1-5, most preferably 1-3;
n indicates the total number of monomers in the polymer backbone and is at least 1, more preferably at least 5, most preferably at least 10.

Since y is independently selected for each monomer unit, polymers of the invention may be homopolymers (if for each monomer y is the same) or copolymers (if at least two values of y are chosen for different monomers).

Most preferably n is selected such that the molecular weight of the polymer is from 100-100,000, more preferably 500-50,000, most preferably 1,000 to 10,000.

Particularly preferred end-groups of the perfluoropolyether (PFPE) material are those wherein z is 1 or 2.

Suitable monomer units for use in polymers of the invention are for example disclosed in EP 360 292. Particularly preferred polymer backbone monomers are of the group consisting of:
a) (CF₂-CF₂-O)
b) (CF₂-O)
c) C₃F₆-O)
d) 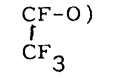
e) 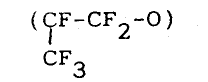
f) (CF₂-O-CF₂-O)
g) (CF₂-O-C₂F₄-O);
and mixtures of these monomers.

Particularly preferred polymers comprise a combination of branched polymer unit, for example monomers d) and/or e), with linear monomers, for example a)-c), f) or g). Especially suitable are polymers comprising mixtures of isopropylether groups and methyl ether groups.

Especially preferred examples of PFPE materials are those having the formula:
wherein the ratio of n to m is from 20 to 40, and wherein preferably the backbone monomers are randomly distributed along the PFPE chain.

Preferred PFPE materials of this formula are those sold under the trade name FOMBLIN HC by Montefluos. For example, FOMBLIN HC/04 (average molecular weight 1500), FOMBLIN HC/25 (average molecular weight 3200) and FOMBLIN HC/R (average molecular weight 6600).

Other suitable materials are sold under the Demnam trade name ex Daikin Industries Ltd, for example Demnam S-20 having a molecular weight of 2,500, Demnam S-65 having a molecular weight of 4,500, Demnam S-100 having a molecular weight of 5,600 and Demnam S-200 having a molecular weight of 8,400.

If mixtures of backbone monomers are used, preferably the different types of monomers are randomly distributed along the PFPE chain.

The level of PFPE material(s) in hair treatment compositions of the invention is from 0.00001 to 0.01% by weight of the composition, preferably from about 0.00005 to about 0.008%, more preferably from about 0.0001 to about 0.005%.

If less than 0.00001% by weight of PFPE is used in the composition, no appreciable improvement in condition will be observed. The use of PFPE levels over 0.01% by weight of the composition provides cost disadvantages, and the hair may appear oily or dull.

### Pearlescing Agent

The pearlescing agent may be selected from a wide range of pearlescing agents. Such pearlescing agents may be selected from C₁₆-C₂₂ fatty acids, C₁₆-C₂₂ esters of fatty acid with alcohols and C₁₆-C₂₂ esters of fatty acids incorporating such elements as alkylene glycol units and the like. Suitable alkylene glycol units may include ethylene glycol and propylene glycol. However, higher alkylene chain length glycols may be employed. Suitable higher alkylene chain length glycols include polyethylene glycol and polypropylene glycol and the like. Preferably, the pearlescing agent may be selected from a wide range of pearlescing agents such as polyethylene glycol mono or diesters of C₁₆-C₂₂ fatty acids having from 1 to 7 ethylene oxide units.

Suitable C₁₆-C₂₂ long chain acyl acids include fatty acids such as stearic acid and behenic acid.

Alternatively, the pearlescing agent may be a long chain acyl derivative material or a mixture of such materials. Ethylene glycol esters of fatty acids having from about 16 to 22 carbon atoms may be suitable.

The pearlescing agent may be a polyethylene glycol mono or diester such as a member selected from the group stearates, oleates, or myristates. Preferably the polyethylene glycol stearate is a distearate.

Preferred esters include polyethylene glycol distearates and ethylene glycol distearates. Examples of a polyethylene glycol distearate available commercially are those of Euperlan PK 900 (ex Henkel) or Genapol TS (ex Hoechst). An example of an ethylene glycol distearate is Euperlan PK 810 (ex Henkel).

Crystals of the pearlescing agent may have a thin platelet shape, and when these crystals are dispersed in the hair treatment composition of the invention, they can help to suspend dispersed particles or droplets by so-called "hindered settling". This contributes to the pearlescent effect observed with such pearlescing agents.

Pearlescing agents must therefore have the following characteristics -
(a) they must have higher dissolution and melting points than normal hair care composition storage temperatures (if the crystals dissolve or melt, then the suspending ability is lost);
(b) they must form thin, platelet-type crystals in the composition.

Monomeric ethylene glycol mono- and di-stearates have been used to suspend particles (e.g. EP 181 773 and EP 34846, both Procter & Gamble). In order to make these shampoos it is necessary to heat the mixture of pearlescing agent (A) and surfactant (B) to above the melting/dissolution point of (A) and (B), and then slowly cool the resultant emulsion whereby platelets of pearlescing agent form.

As a further alternative, the pearlescing agent may be a long chain acyl derivative material or mixture of such materials. Such materials are described in EP 285388 (to Procter and Gamble) and include the ethylene glycol esters of fatty acids having from about 16 to about 22 carbon atoms.

Included are ethylene glycol esters of fatty acids having from about 16 to about 22 carbon atoms. Preferred are the ethylene glycol stearates, both mono and distearate, but particularly the distearate containing less than about 7% of the mono stearate. Other pearlescing agents include alkanol amides of fatty acids, having from about 16 to about 22 carbon atoms, preferably about 16 to 18 carbon atoms. Preferred alkanol amides are stearic monoethanolamide, stearic diethanolamide, stearic monoisopropanolamide and stearic monoethanolamide stearate. Other long chain acyl derivatives include long chain esters of long chain fatty acids (e.g. stearyl stearate, cetyl palmitate etc.); glyceryl esters (e.g. glyceryl distearate) and long chain esters of long chain alkanol amides (e.g. stearamide DEA distearate, stearamide MEA stearate).

Further suitable pearlescing agents include alkyl (C₁₈₋₂₂) dimethyl amine oxides such as stearyl dimethyl amine oxide. If the compositions contain an amine oxide or a long chain acyl derivative as a surfactant the suspending function could also be provided and additional suspending agent may not be required if the level of those materials are at least the minimum level given below.

The pearlescing agent may be present in an amount of from about 0.005% to about 5.0% by weight. Preferably the pearlescing agent is present in an amount of from about 0.1% to about 3% by weight.

### OTHER INGREDIENTS

Preferred hair treatment compositions of the invention comprise one or more surfactant materials and/or one or more conditioning agents.

Shampoo compositions in accordance with the present invention preferably comprise one or more surfactant materials selected from anionic, nonionic, amphoteric, zwitterionic or cationic surfactants or mixtures thereof.

Hair conditioning shampoos may also comprise one or more cationic surfactants. The use of cationic surfactants is especially preferred, because these ingredients are capable of providing conditioning benefits to hair.

Suitable anionic surfactants are the alkyl sulphates, alkyl ether sulphates, alkaryl sulphonates, alkyl succinates, alkyl sulphosuccinates, N-alkoyl sarcosinates, alkyl phosphates, alkyl ether phosphates, alkyl ether carboxylates, and a-olefin sulphonates, especially their sodium, magnesium, ammonium and mono-di and tri-ethanolamine salts. The alkyl groups generally contain from 8 to 18 carbon atoms and may be unsaturated. The alkyl ether sulphates, alkyl ether phosphates and alkyl ether carboxylates may contain from 1 to 10 ethylene oxide or propylene oxide units per molecule, and preferably contain 2 to 3 ethylene oxide units per molecule.

Examples of suitable anionic surfactants include sodium oleyl succinate, ammonium lauryl sulphosuccinate, ammonium lauryl sulphate, sodium dodecylbenzene sulphonate, triethanolamine dodecylbenzene sulphonate and sodium N-lauryl sarcosinate. The most preferred anionic surfactants are sodium lauryl sulphate, triethanolamine lauryl sulphate, triethanolamine monolauryl phosphate, sodium lauryl ether sulphate 1EO, 2EO and 3EO, ammonium lauryl sulphate and ammonium lauryl ether sulphate 1EO, 2EO and 3EO.

The nonionic surfactants suitable for use in the composition of the invention may include condensation products of aliphatic (C₈-C₁₈) primary or secondary linear or branched-chain alcohols or phenols with alkylene oxides, usually ethylene oxide and generally 6-30 EO.

Other suitable nonionics include mono- or dialkyl alkanolamides or alkyl polyglucosides. Examples include coco mono- or di-ethanolamide, coco monoisopropanolamide, and coco di-glucoside.

Amphoteric and zwitterionic surfactants suitable for use in the composition of the invention may include alkyl amine oxides, alkyl betaines, alkyl amidopropyl betaines, alkyl sulphobetaines, alkyl glycinates, alkyl carboxyglycinates, alky amphopropionates, alkyl amidopropyl hydroxysultaines, acyl taurates and acyl glutamates wherein the alkyl and acyl groups have from 8 to 19 carbon atoms. Examples include lauryl amine oxide, cocodimethyl sulphopropyl betaine and preferably lauryl betaine, cocoamidopropyl betaine and sodium cocamphropionate.

Examples of cationic surfactants include: cetyl trimethylammonium chloride, stearyl dimethylbenzyl ammonium chloride, cetylpyridinium chloride, Quaternium-5, -31, -18, and mixtures thereof.

The level of surfactant material(s) in shampoo compositions of the invention is preferably more than 1%, more preferably 2-35% and most preferably from 5 to 30% by weight of the composition. In conditioning shampoos according to the invention the level of cationic surfactants may be of from about 0.01 to about 10%, more preferably, 0.05 to 5%, most preferably 0.1 to 2% by weight of the composition.

If the hair-treatment composition of the invention comprises in addition to the perfluoropolyether materials and the cationic surfactant -if any- an additional conditioning agent, this material is preferably chosen from cationic polymers, volatile and non-volatile silicones, protein hydrolysates or quaternised protein hydrolysates.

Suitable cationic polymers include Guar hydroxypropyltrimonium chloride, Quaternium-19, 23, -40, -57, poly (dimethyldiallylammonium chloride), poly (dimethyl butenyl ammonium chloride)-, w- bis (triethanolammonium chloride), Poly (dipropyldiallylammonium chloride), Poly (methyl-beta-propaniodiallylammonium chloride), Poly (diallylpiperidinium chloride), poly (vinyl pyridinium chloride), quaternised poly (vinyl alcohol), quaternised poly (dimethylaminoethylmethacrylate) and mixtures thereof.

Examples of suitable volatile silicone materials include cyclomethicone, available commercially as Dow Corning DC 345, and Volatile Silicone 7158, available from Union Carbide.

Suitable protein derivatives include lauryl dimonium hydroxy propylamino hydrolysed animal protein, available commercially under the tradename LAMEQUAT L, and hydrolysed keratin containing sulphur-bearing amino acids, available commercially under the tradename CROQUAT WKP.

Conditioning agents which are especially suitable for use in compositions according to the invention include volatile and non-volatile silicone oils, such as for example polyalkylsiloxanes, polyalkylaryl siloxanes, silicone gums, cyclomethicones and aminofunctional silicones.

The preferred level of conditioning agents other than perfluoropolyethers and cationic surfactants in compositions of the invention is from 0 to 20%, for example from 0.01 to 10% or from 0.1 to 5% by weight.

Another ingredient that may if desired be incorporated into compositions of the invention is a fatty alcohol material. The use of this material is especially preferred in conditioning compositions of the invention, in particular conditioning shampoo compositions which comprise one or more cationic surfactants. The combined use of fatty alcohol material and cationic surfactant is believed to be advantageous, because this leads to the formation of a lamellar phase wherein the cationic surfactant is stably dispersed.

Preferred fatty alcohols comprise from about 8 to about 22 carbon atoms, more preferably 16 to 20. Examples of preferred fatty alcohols include cetyl alcohol and stearyl alcohol. The use of these materials is also advantageous in that they contribute to the overall conditioning properties of compositions of the invention.

The level of fatty alcohol material(s), if used, is preferably from 0 to 10%, more preferably from 0.1 to 5% by weight of the composition. The weight ratio of cationic surfactant to fatty alcohol is suitably from 10 : 1 to 1 : 10, more preferably from 4 : 1 to 1 : 8, most preferably from 1 : 1 to 1 : 4.

### Minor Ingredients

The hair-treatment compositions of the invention may also include minor amounts of one or more other ingredients commonly found in hair-treatment compositions, such as antibacterial agents, antidandruff agents such as zinc pyridinethione or Octopirox, foam boosters, perfumes, dyes, colouring agents, preservatives, viscosity modifiers, proteins, polymers, buffering agents, polyols and other moisturising agents, herb extracts, mink oil or honey.

### Water

Compositions of the invention preferably comprise from 20-99.5% of water, more preferably 60-98%, most preferably 80-96% by weight.

### USE OF THE COMPOSITION

After preparation, hair treatment compositions according to the invention are preferably packed. Any suitable container can be used for this purpose, but generally compositions of the invention will be packed in closed containers like bottles, sachets and the like.

Hair treatment compositions of the invention are generally applied in an amount of from 1 to 50mls. Preferred amounts for shampoos are 3 to 10mls to wet hair, preferably from 3 to 5 mls to wet hair. After applying the shampoo the wet hair is worked to create a lather. The latter may be retained on the head for a short time before rinsing, e.g. from 1 to 4 minutes, or may immediately be rinsed off. The treatment may be repeated, if required. For conditioners the preferred dosage is from 5 to 20mls which is applied to hair after washing or rinsing, whereafter the wet hair is worked and rinsed.

In preparing hair-treatment compositions of the invention, preferably conventional hot or cold mixing processes are used. Preferred methods for adding the PFPE ingredient to the compositions are the forming of a predispersion of PFPE with small amounts of surfactants in combination with a thickening amount of electrolyte, or a predispersion can be formed of PFPE with polymer ingredients, or a predispersion of PFPE with low levels of surfactant in combination with glycerol. This predispersion is then used in the preparation of the final hair-treatment composition.

The invention is further illustrated by the following Examples:

### Example 1

| Component | % by weight |
|---|---|
| Sodium lauryl ether sulphate 2EO | 12 |
| Cocamidopropyl betaine | 4 |
| Jaguar C13S | 0.2 |
| Perfluoropolyether (1) | 0.0003 |
| NaCl | 1.0 |
| Ethylene glycol distearate | 2.0 |
| Preservative, perfume,colour | q.s. |
| Water | to 100 |

| | |
|---|---|
| (1) Demnam S-20, S-65, S-100 or S-200 Fomblin HC/04, HC/25 or HC/R | |

### Example 2

| Component | % by weight |
|---|---|
| Sodium lauryl ether sulphate 2EO | 12 |
| Cocoamidopropyl betaine | 4 |
| Jaguar C13S | 0.2 |
| Perfluoropolyether (1) | 0.003 |
| NaCl | 1.0 |
| Ethylene glycol distearate | 0.5 |
| Preservative, perfume, colour | q.s. |
| Water | to 100 |

| | |
|---|---|
| (1) As per example 1 | |

### Example 3

| Component | % by weight |
|---|---|
| Sodium lauryl ether sulphate 2EO | 16 |
| Lauryl betaine | 2 |
| Jaguar C13S | 0.15 |
| Polyethylene glycol (3EO) distearate | 2.0 |
| Perfluoropolyether (1) | 0.001 |
| NaCl | 1.0 |
| Preservative, perfume, colour | q.s. |
| Water | to 100 |

| | |
|---|---|
| (1) As per example 1 | |

### Example 4

| Component | % by weight |
|---|---|
| Ammonium lauryl sulphate | 12.0 |
| Lauryl betaine | 2.0 |
| Ethylene glycol distearate | 2.0 |
| Perfluoropolyether (1) | 0.005 |
| Coconut diethanolamide | 1.0 |
| Preservative, perfume, colour | q.s. |
| Water | to 100 |

| | |
|---|---|
| (1) As per example 1 | |

### Example 5

| Component | % by weight |
|---|---|
| Triethanolamine lauryl sulphate | 12 |
| Jaguar HP60 | 1.0 |
| Ethylene glycol distearate | 2.0 |
| Perfluoropolyether (1) | 0.003 |
| Preservative, perfume, colour | q.s. |
| Water | to 100 |

| | |
|---|---|
| (1) As per example 1 | |

There now follows a comparative example wherein formulations of the instant invention are tested against formulations of conventional non-pearlescing systems.

### Comparative Example

- A: = Control
- B: = Non-pearlescing example of a formulation including perfluoropolyether
- C: = Formulation of the invention
- D: = Pearlescing formulation excluding perfluoropolyether as a component

| | A | B | C | D |
|---|---|---|---|---|
| SLES 2EO | 12.0 | 12.0 | 12.0 | 12.0 |
| Cocoamido propyl betaine | -- | 4.0 | 4.0 | 4.0 |
| Jaguar C13S | -- | 0.2 | 0.2 | 0.2 |
| Perfluoropolyether | -- | 0.0003 | 0.0003 | -- |
| Ethylene glycol distearate | -- | -- | 0.25 | 0.25 |
| Colour, perfume preservative | qs | qs | qs | qs |
| Water | to 100 | to 100 | to 100 | to 100 |

### In-Vitro Testing

12g of hair in the form of a swatch was worked in 1.2g of shampoo, lathered for 30 seconds, left for 20 seconds, and rinsed with water. The procedure was repeated once. The hair was then dried. Three (3) switches of hair were prepared for each product to be evaluated.

The evaluation was carried out by twelve (12) trained assessors in the form of paired comparison test. The results were interpreted statistically at 0.01 and 0.05 confidence levels.

Softness was assessed by feel.

Shine was assessed by viewing the switches mounted over a curved surface under a spotlight to create a highlight which was then visually assessed for its light intensity.

The results are shown in Table 1 below.

**Table 1**

| Control | V | Test | Softness | Shine |
|---|---|---|---|---|
| | | | [% of votes for test (at indicated significance level)] | |
| A | V | B | 57 (n.s.)¹ | 64 (0.05) |
| A | V | C | 71 (0.01) | 67 (0.05) |
| A | V | D | 63 (0.05) | 44 (n.s.) |

| | | | | |
|---|---|---|---|---|
| ¹n.s. = not significant | | | | |

From the results it can be seen that formulation C has greater softness and shine relative to control formulation A than the softness and shine of formulations B and D relative to control formulation A. It is clear that formulation C provides for superior shine and softness benefit relative to formulations B and D.

## Claims

1. A hair treatment composition comprising:
(i) from 0.00001 to 0.01% by weight of a perfluoropolyether material; and
(ii) a suitable pearlescing agent, characterised by:
(a) having higher dissolution and melting points than normal hair care composition storage temperatures; and (b) forming thin platelet-type crystals in the hair treatment composition.

2. A hair treatment composition according to claim 1, wherein the perfluoropolyether material is present in an amount of from 0.0001 to 0.005% by weight.

3. A hair treatment composition according to claim 1 or claim 2, wherein the perfluoropolyether is of the formula:
F-(C_{y}F_{2y}O)ₙ-C_{z}F_{(2z+1)}
wherein
z is an integer from 1 to 6;
for each monomer, y is independently selected from the integer-range from 1 to 6;
n indicates the total number of monomer units in the polymer backbone and is at least 1.

4. A hair treatment composition according to claim 3, wherein n is such that the molecular weight of the perfluoropolyether is from 100 to 100,000.

5. A hair treatment composition according to claim 1 or claim 2, wherein the perfluoropolyether is of the formula: 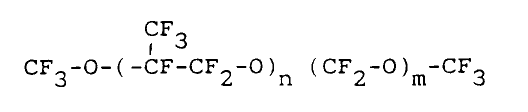 wherein the ratio of n to m is from 20 to 40.

6. A hair treatment composition according to any preceding claim, wherein the pearlescing agent is selected from the group C₁₆-C₂₂ fatty acids C₁₆-C₂₂ esters of fatty acids with alcohols and C₁₆-C₂₂ esters of fatty acids incorporating alkylene glycol units.

7. A hair treatment composition according to claim 6, wherein the alkylene glycol units comprise polyethylene glycol or polypropylene glycol.

8. A hair treatment composition according to claim 6 wherein the pearlescing agent is selected from ethylene or polyethylene glycol mono- or di-esters of C₁₆-C₂₂ fatty acids having from 1 to 7 ethylene oxide units.

9. A hair treatment composition according to any preceding claim which is a shampoo, the composition further comprising at least 1% by weight of one or more surfactants.

10. A hair treatment composition according to any preceding claim which is a conditioning shampoo, the composition further comprising from 0.01 to 10% by weight of one or more cationic surfactants.

11. A hair treatment composition according to any preceding claim which is a conditioner, the composition further comprising up to 20% by weight of one or more additional conditioning agents.

12. Use of a composition according to any preceding claim for the treatment of hair.

13. A method of conditioning hair comprising applying thereto a composition comprising:
(i) from 0.00001 to 0.01% by weight of a perfluoropolyether material; and
(ii) a suitable pearlescing agent, characterised by:
(a) having higher dissolution and melting points than normal hair care composition storage temperatures; and
(b) forming thin platelet-type crystals in the hair treatment composition.

## Patentansprüche

1. Haarbehandlungszusammensetzung, die
(i) 0,00001 bis 0,01 Gew.-% eines Perfluorpolyether-materials und
(ii) ein geeignetes, permuttglänzendes Mittel umfaßt, dadurch gekennzeichnet, daß sie
(a) einen über der Lagerungstemperatur normaler Haarpflegezusammensetzungen liegenden Auflösungs- und Schmelzpunkt aufweist und
(b) dünne plättchenartige Kristalle in der Haarbehandlungszusammensetzung ausbildet.

2. Haarbehandlungszusammensetzung nach Anspruch 1, wobei das Perfluorethermaterial in einer Menge von 0,0001 bis 0,005 Gew.-% vorhanden ist.

3. Haarbehandlungszusammensetzung nach Anspruch 1 oder Anspruch 2, wobei der Perfluorpolyether ein solcher der folgenden Formel ist:
F-(C_{y}F_{2y}O)ₙ-C_{z}F_{(2z+1)}
worin z eine ganze Zahl von 1 bis 6 bedeutet, bei jedem Monomer y unabhängig voneinander unter einem Bereich ganzer Zahlen von 1 bis 6 ausgewählt ist und n die Gesamtzahl der Monomereneinheiten im Polymerrückgrat bedeutet und mindestens 1 ist.

4. Haarbehandlungszusammensetzung nach Anspruch 3, wobei n einen derartigen Wert besitzt, daß das Molekulargewicht des Perfluorpolyethers zwischen 100 und 100 000 liegt.

5. Haarbehandlungszusammensetzung nach Anspruch 1 oder Anspruch 2, wobei der Perfluorpolyether ein solcher der folgenden Formel ist: 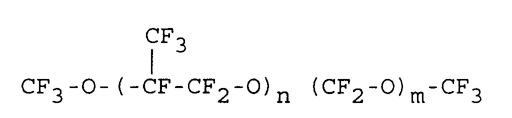 worin das Verhältnis n/m 20 bis 40 beträgt.

6. Haarbehandlungszusammensetzung nach einem der vorhergehenden Ansprüche, wobei das permuttglänzende Mittel unter C₁₆-C₂₂-Fettsäuren, C₁₆-C₂₂-Estern von Fettsäuren mit Alkoholen und C₁₆-C₂₂-Estern von Fettsäuren, die Alkylenglykoleinheiten enthalten, ausgewählt ist.

7. Haarbehandlungszusammensetzung nach Anspruch 6, wobei die Alkylenglykoleinheiten Polyethylenglykol oder Polypropylenglykol umfassen.

8. Haarbehandlungszusammensetzung nach Anspruch 6, wobei das permuttglänzende Mittel unter Ethylen- oder Polyethylenglykolmono- oder -diestern von C₁₆-C₂₂-Fettsäuren mit 1 bis 7 Ethylenoxideinheiten ausgewählt ist.

9. Haarbehandlungszusammensetzung nach einem der vorhergehenden Ansprüche, bei der es sich um ein Shampoo handelt, wobei die Zusammensetzung des weiteren mindestens 1 Gew.-% eines oder mehrerer grenzflächenaktiver Mittel umfaßt.

10. Haarbehandlungszusammensetzung nach einem der vorhergehenden Ansprüche, bei der es sich um ein konditionierendes Shampoo handelt, wobei die Zusammensetzung des weiteren 0,01 bis 10 Gew.-% eines oder mehrerer kationischer grenzflächenaktiver Mittel umfaßt.

11. Haarbehandlungszusammensetzung nach einem der vorhergehenden Ansprüche, bei der es sich um ein Konditioniermittel handelt, wobei die Zusammensetzung des weiteren bis zu 20 Gew.-% eines oder mehrerer weiterer konditionierender Mittel umfaßt.

12. Verwendung einer Zusammensetzung nach einem der vorhergehenden Ansprüche zur Behandlung von Haar.

13. Verfahren zur Konditionierung von Haar durch Applizieren einer Zusammensetzung, die
(i) 0,00001 bis 0,01 Gew.-% eines Perfluorpolyethermaterials und
(ii) ein geeignetes, permuttglänzendes Mittel umfaßt, dadurch gekennzeichnet, daß sie
(a) einen über der Lagerungstemperatur normaler Haarpflegezusammensetzungen liegenden Auflösungs- und Schmelzpunkt aufweist und
(b) dünne plättchenartige Kristalle in der Haarbehandlungszusammensetzung ausbildet.

## Revendications

1. Une composition pour le traitement des cheveux contenant :
(i) une matière de perfluoropolyéther selon des proportions comprises entre environ 0,00001 et environ 0,01 % en masse ; et
(ii) un agent nacre approprié, caractérisée en ce qu'elle :
(a) présente des points de dissolution et de fusion plus élevés que la température de stockage d'une composition pour le traitement des cheveux ordinaire ; et
(b) forme des cristaux fins de type plaquettes dans la composition pour le traitement des cheveux.

2. Une composition pour le traitement des cheveux selon la Revendication 1, dans laquelle la matière de perfluoropolyéther est présente dans des proportions comprises entre 0,0001 et 0,005 % en masse.

3. Une composition pour le traitement des cheveux selon la Revendication 1 ou 2, dans laquelle le perfluoropolyéther présente la formule suivante :
F-(C_{y}F_{2y}O)ₙ-C_{z}F _{(2z+1)}
où z est un entier compris entre 1 et 6 ;
pour chaque monomère, y est indépendamment sélectionné parmi la gamme d'entiers compris entre 1 et 6 ;
n indique le nombre total d'unités monomères présentes dans le squelette de polymère et est au moins égal à 1.

4. Une composition pour le traitement des cheveux selon la Revendication 3, dans laquelle n est tel que la masse moléculaire du perfluoropolyéther est comprise entre 100 et 100 000.

5. Une composition pour le traitement des cheveux selon la Revendication 1 ou 2, dans laquelle le perfluoropolyéther présente la formule suivante : 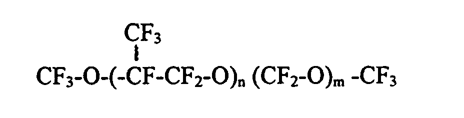 où le rapport entre n et m est compris entre 20 et 40.

6. Une composition pour le traitement des cheveux selon l'une quelconque des revendications précédentes, dans laquelle l'agent nacre est sélectionné parmi le groupe composé d'acides gras en C₁₆-C₂₂, d'esters en C₁₆-C₂₂ d'acides gras avec des alcools, et d'esters en C₁₆-C₂₂ d'acides gras incorporant des unités d'alkylèneglycol.

7. Une composition pour le traitement des cheveux selon la Revendication 6, dans laquelle les unités d'alkylèneglycol comprennent du polyéthylèneglycol ou du polypropylèneglycol.

8. Une composition pour le traitement des cheveux selon la Revendication 6, dans laquelle l'agent nacre est sélectionné parmi des mono- ou diesters de polyéthylène- ou d'éthylèneglycol d'acides gras en C₁₆-C₂₂ présentant 1 à 7 unités d'oxyde d'éthylène.

9. Une composition pour le traitement des cheveux selon l'une quelconque des Revendications précédentes, qui est un shampooing, la composition comprenant en outre au moins 1 % en masse d'un ou de plusieurs agent(s) tensioactif(s).

10. Une composition pour le traitement des cheveux selon l'une quelconque des Revendications précédentes, qui est un shampooing de conditionnement, la composition comprenant en outre 0,01 à 10 % en masse d'un ou de plusieurs agent(s) tensioactif(s) cationique(s).

11. Une composition pour le traitement des cheveux selon l'une quelconque des Revendications précédentes, qui est un conditionneur, la composition comprenant en outre jusqu'à 20 % en masse d'un ou de plusieurs agent(s) de conditionnement supplémentaire(s).

12. Utilisation de la composition selon l'une quelconque des Revendications précédentes pour le traitement des cheveux.

13. Une méthode de conditionnement des cheveux comprenant l'application sur la tête d'une composition comprenant :
(i) une matière de perfluoropolyéther selon des proportions comprises entre 0,00001 et 0,01 % en masse ; et
(ii) un agent nacre approprié, caractérisée en ce qu'elle :
(a) présente des points de dissolution et de fusion plus élevés que la température de stockage d'une composition pour le traitement des cheveux ordinaire ; et
(b) forme des cristaux fins de type plaquettes dans la composition pour le traitement des cheveux.
